# EUROPEAN PATENT APPLICATION

(11) **EP 4 289 343 A1**
(43) Date of publication of application: **13.12.2023**
(21) Application number: 22382552.2
(22) Date of filing: 09.06.2022
(51) Int. Cl.: A61B 5/00, A61B 5/05, A61B 18/02, A61B 18/00, G06F 9/50

(54) **ASSESSING ABLATION LESIONS IN REALTIME**

(71) Applicant: Medlumics S.L., 28760 Tres Cantos, Madrid (ES)
(72) Inventor: Sancho, Juan, 28760 Plaza de la Encina 10-11, Núcleo 3, 2°A Tres Cantos - Madrid (ES); Herranz, David, 28760 Plaza de la Encina 10-11, Núcleo 3, 2°A Tres Cantos - Madrid (ES); Bailleul, Christophe, 28760 Plaza de la Encina 10-11, Núcleo 3, 2°A Tres Cantos - Madrid (ES); Greene, James, 28760 Plaza de la Encina 10-11, Núcleo 3, 2°A Tres Cantos - Madrid (ES)
(74) Representative: DTS Patent- und Rechtsanwälte Schnekenbühl und Partner mbB

(57) **Abstract**

Disclosed herein are system, method, and computer-readable medium aspects for assessing ablation lesions in realtime. An aspect operates by receiving a first optical measurement data from a first catheter optical port, assigning the first optical measurement data to a first available processing core in a processing unit in order to identify an optical property at a first location of a lesion, and generating a first graphical representation from the optical property at the first location of the lesion. After a predetermined time, the aspect continues to operate by repeating the receiving, assigning, and generating operations for a second optical measurement data using a second available processing core in order to generate a second graphical representation from the optical property at a second location of the lesion. The aspect concludes by displaying the first graphical representation and the second graphical representation on a user interface at a predefined interval.

## Description

### BACKGROUND

### Technical Field

Aspects of the present disclosure relate to components, systems, and methods for using catheter and console devices for optical signal analysis, reducing processing latency, assessing ablation lesions, and estimating lesion depths.

### Background

Ablation is a medical technique for producing tissue necrosis. It is used to help treat different pathologies including cancer, Barret's esophagus, renal denervation, pulmonary denervation or cardiac arrhythmias, among others. Various energy sources may be utilized for ablation. For example, in radiofrequency (RF) ablation, an external electrode is placed on a patient's body, and an alternating potential is applied to the tip of a catheter that is placed in contact with the tissue to be treated within the patient's body. The application of alternating current with an oscillating frequency above several hundreds of kHz avoids the stimulation of excitable tissue while delivering heat by means of the Joule's effect. The increase in tissue temperature produces denaturation of the biological molecules, including proteins such as collagen, myosin, or elastin.

### SUMMARY

In aspects presented herein, optical systems, processing devices, and catheters may provide optical measurements for understanding optical properties, such as birefringence, tissue anisotropy, polarization, and/or phase retardation of tissue, in real-time in order to monitor changes in the optical properties over time and estimate lesion depths in the tissue.

In an aspect, an example method comprises receiving an optical measurement data from a catheter optical port and assigning the optical measurement data to be processed in an available processing core of a processing unit. The method then comprises directing the available processing core to implement a processing chain on the optical measurement data in order to identify an optical property at a location of a lesion and generating a graphical representation from the optical property at the location of the lesion. After a predetermined time or the time of ablation or at any time during and throughout the application of energy used for tissue ablation, such as radiofrequency, irreversible electroporation, cryotherapy, ultrasound, or laser the method further comprises repeating the outlined steps for a second optical measurement data using a second available processing core in order to generate a second graphical representation. The method then concludes by displaying the graphical representations on a user interface at a predefined interval. Can we say throughout the ablation procedure?

In another aspect, an example system is described. The system comprises a catheter comprising a first catheter optical port and a second catheter optical port, a computing device coupled to the catheter and comprising a processor and a memory, and a user interface coupled to the computing device. The processor further comprises a processing unit and the memory contains instructions that when executed by the processor cause the computing device to receive a first optical measurement data from the first catheter optical port, assign the first optical measurement data to a first available processing core in the processing unit, direct the first available processing core to implement a processing chain on the first optical measurement data in order to identify an optical property at a first location of a lesion, and to generate a first graphical representation from the optical property at the first location. After a predetermined time, the computing device repeats the outlined steps for a second optical measurement data using the second catheter optical port and a second available processing core in order to generate a second graphical representation. The processor then causes the computing device to display the first and second graphical representations on the user interface at a predefined interval.

In yet another aspect, an example non-transitory computer-readable medium has instructions stored on it that, when executed by at least one computing device, cause the at least one computing device to perform operations. The operations comprise receiving an optical measurement data from a catheter optical port and assigning the optical measurement data to be processed in an available processing core of a processing unit. The operations further comprise directing the available processing core to implement a processing chain on the optical measurement data in order to identify an optical property at a location of a lesion, and generating a graphical representation from the optical property at the location of the lesion. After a predetermined time, the operations comprise repeating the outlined steps for a second optical measurement data using a second available processing core in order to generate a second graphical representation. The operations then conclude by displaying the graphical representations on a user interface at a predefined interval.

Further features and advantages, as well as the structure and operation of various aspects, are described in detail below with reference to the accompanying drawings. It is noted that the specific aspects described herein are not intended to be limiting. Such aspects are presented herein for illustrative purposes only. Additional aspects will be apparent to persons skilled in the relevant art(s) based on the teachings contained herein.

### BRIEF DESCRIPTION OF THE DRAWINGS

The accompanying drawings, which are incorporated herein and form a part of the specification, illustrate aspects of the present disclosure and, together with the description, further serve to explain the principles of the disclosure and to enable a person skilled in the pertinent art to make and use the disclosure.
FIG. 1 is a diagram of a system for assessing ablation lesions in real-time, according to some aspects of the present disclosure.
FIG. 2 is a diagram of a catheter, according to some aspects of the present disclosure.
FIG. 3 is a block diagram of a processing unit, according to some aspects of the present disclosure. Still relevant or has this evolved with the new console, laser?
FIG. 4 is a diagram of a user interface, according to some aspects of the present disclosure. Update with latest design
FIG. 5 is a timing diagram of timing for assessing ablation lesions in realtime, according to some aspects of the present disclosure.
FIG. 6 is a block diagram of a processing chain, according to some aspects of the present disclosure. Still correct or needs updating?
FIG. 7 is a flowchart of a method for assessing ablation lesions in realtime, according to some aspects of the present disclosure.
FIG. 8 is a block diagram of an example computer system useful for implementing various aspects. Still relevant? Needs updating?

In the drawings, like reference numbers generally indicate identical or similar elements. Additionally, generally, the left-most digit(s) of a reference number identifies the drawing in which the reference number first appears.

Aspects of the present disclosure will be described with reference to the accompanying drawings.

### DETAILED DESCRIPTION

Provided herein are system, apparatus, device, method and/or computer-readable medium aspects, and/or combinations and sub-combinations thereof for assessing ablation lesions in realtime.

Denaturation of biological molecules creates a lesion on the tissue. Assessing characteristics of the lesion is important to producing tissue necrosis sufficient to treat different pathologies without damaging healthy tissue. The lesion can be assessed using optical ports located at the tip of the catheter and various optical data processors. Because of latency in processing lesion characteristics, it may be difficult to understand ablation effects in tissue and to avoid damaging healthy tissue using current systems and methods. Current systems and methods for assessing ablation lesions do not achieve the level of realtime processing necessary to prevent improper denaturation of biological molecules due to processing latency. Improper denaturation occurs where a lesion is created on a tissue in order to treat different pathologies but where healthy tissue is damaged due to an over-ablation of the tissue, or where the tissue is not sufficiently ablated.

Aspects herein solve this technological problem using an innovative parallel processing approach using a multi-core processor and a processing chain. A multi-port optical component may be used to transmit data from different tissue sites through a catheter to a console for processing, each port being associated with a different viewing angle and/or portion of tissue. In such a multi-port optical component, only one optical interrogation port may actively provide data at a given time. To obtain data from an entire tissue site, even though only one optical interrogation port may be active at a given time, data may be received from all the optical interrogation ports by cycling through each port output. Once sufficient data has been received from one optical interrogation port, for example, a data analysis console may switch inputs to a different optical interrogation port.

In a single core processing system, the data from different optical interrogation ports would need to be processed sequentially. Thus, the total amount of processing time needed to generate data across an entire tissue site constitutes the sum total of the time it takes the single core to both receive data from each optical interrogation site, and process the data received from each optical interrogation site. In an implementation where tissue is being ablated based on information obtained from the optical interrogation, a complete picture of the ablation cannot be obtained for that sum total of time.

In a multi-core processing system, however, data inputs can be processed in parallel. However, in a multi-port ablation catheter, data from each optical port is not received at once such that true parallel processing can occur. Rather, data is received from a first optical port, and then from a second optical port once the optical input has been switched. According to aspects of the invention, a first available processing core may implement the processing chain on a first optical measurement data in order to generate a first estimated lesion depth. In parallel but with an offset to account for the amount of time needed to receive a second optical measurement data, a second available processing core may implement the processing chain on the second optical measurement data in order to generate a second estimated lesion depth. The processing core assignments can be timed to synchronize with the switching of optical port inputs. Because the processing cores are processing the optical measurement data in near-parallel, the estimated lesion depths can be displayed on a user interface with little delay.

Aspects herein provide various benefits. For example, the parallel processing approach allows a user to view updated estimated lesion depths on a user interface with little delay, instead of having to wait for a processing system to implement a processing chain consecutively. In other words, while a user is ablating a tissue and producing tissue necrosis to treat different pathologies, the user can quickly assess characteristics of the ablation lesion to ensure that pathologies are treated and healthy tissue is protected. Therefore, the parallel processing approach solves the above technological problem by assessing ablation lesions with realtime processing sufficient to allow the treatment of different pathologies without damaging healthy tissue.

It is noted that although this application may refer specifically to cardiac ablation, the aspects described herein may target other pathologies as well, along with additional energy sources for ablation, including but not limited to cryogenic, radiofrequency (RF), microwave, laser, ultrasound, and pulsed electric fields. The principles of using energy to treat other pathologies are similar, and therefore the techniques used to apply the energy are similar. It is also noted that the aspects described herein may be used in vivo or in vitro.

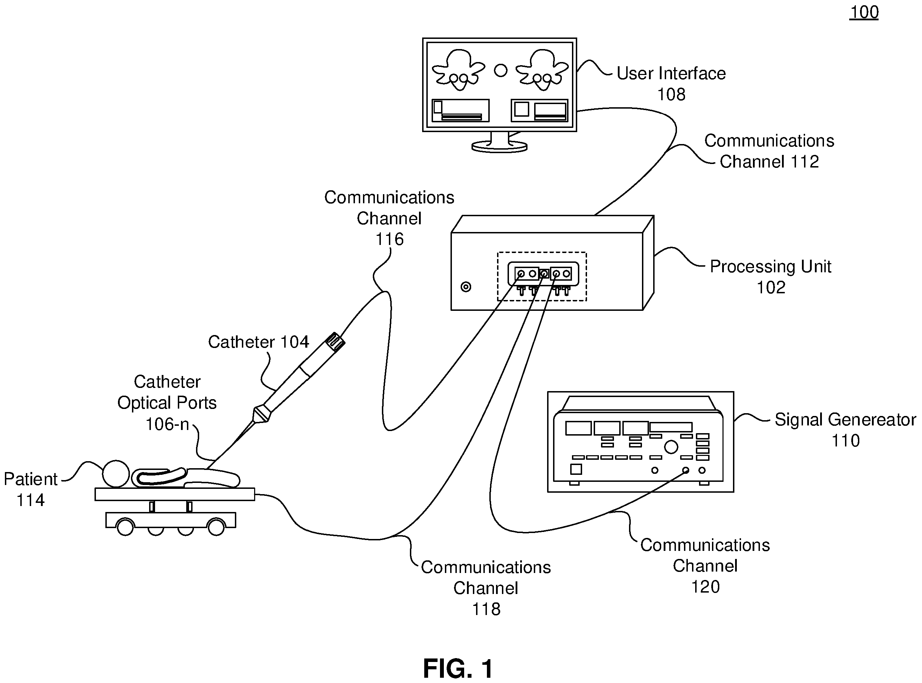

FIG. 1 is a diagram of a system 100 for assessing ablation lesions in realtime, according to some aspects of the present disclosure. System 100 may include processing unit 102, catheter 104, catheter optical ports 106a-n, user interface 108, signal generator 110, patient 114, and communications channels 112/116/118/120.

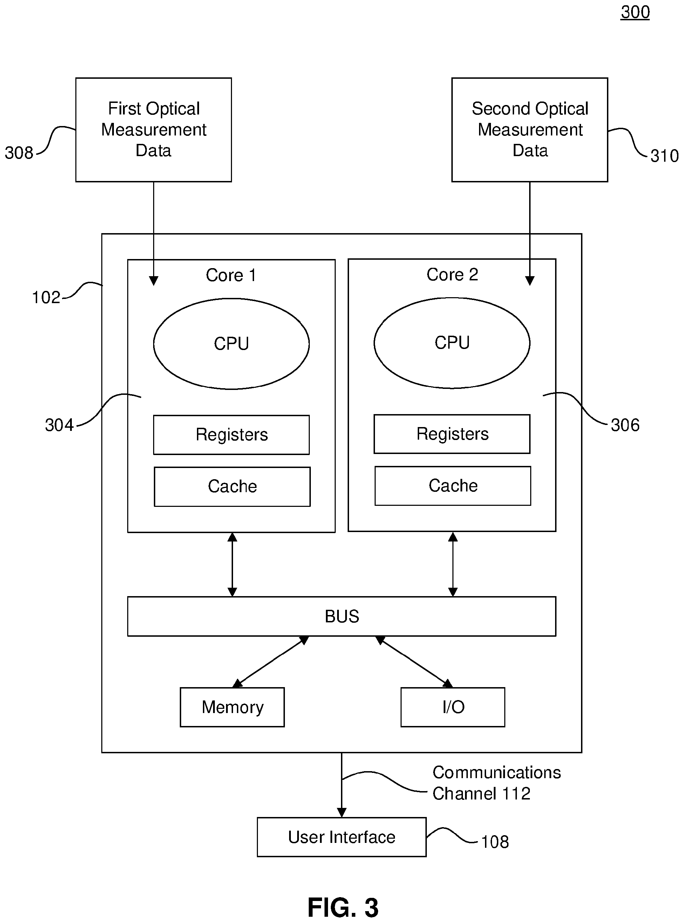

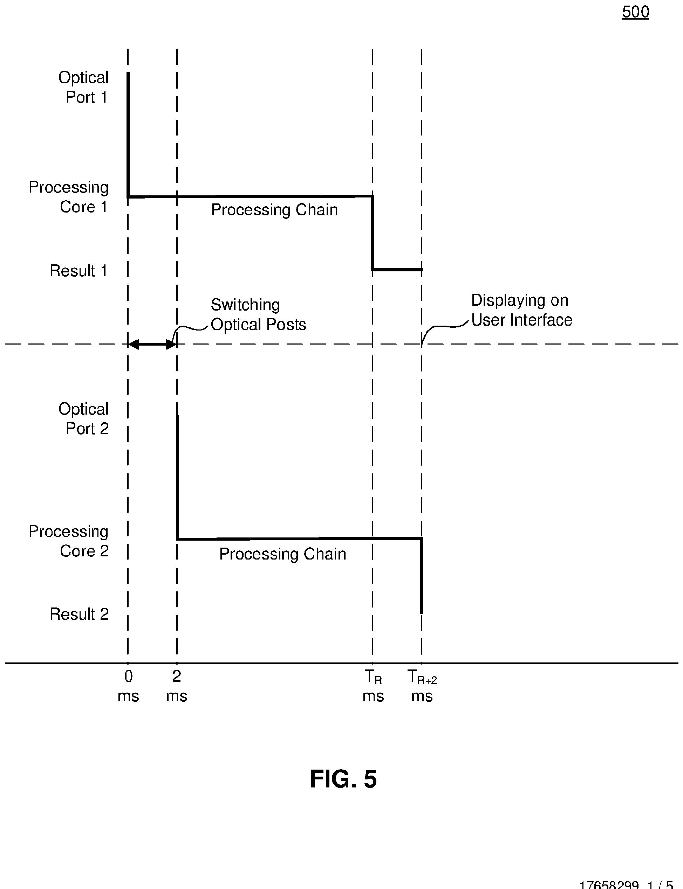

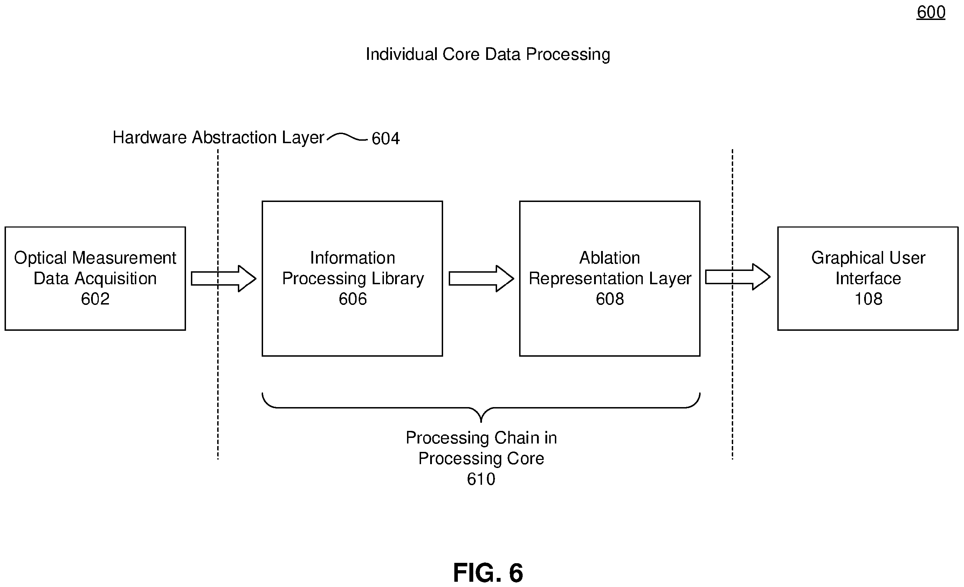

Processing unit 102 is also described below with reference to FIGS. 3, 5, and 6. FIG. 3 is a block diagram of a processing unit 102, according to some aspects of the present disclosure. FIG. 5 is a timing diagram of timing for assessing ablation lesions in realtime, according to some aspects of the present disclosure. FIG. 6 is a block diagram of a processing diagram 600, according to some aspects of the present disclosure.

Processing unit 102 may process optical measurement data collected from catheter optical ports 106a-n in catheter 104. Processing unit 102 may interface with catheter 104 through communications channel 116. Communications channel 116 may be wired, wireless, or a combination thereof. Communications channel 116 may be optical connections or electrical connections. Optical connections may include single mode optical fibers or multimode optical fibers that allow acquisition or transmission of optical signals, and electrical connections may include wiring, pins, or components used for supplying power and energy. Communications channel 116 may include any combination of Local Area Networks, Wide Area Networks, the Internet, etc. Control logic or data may be transmitted to and from processing unit 102 via communications channel 116.

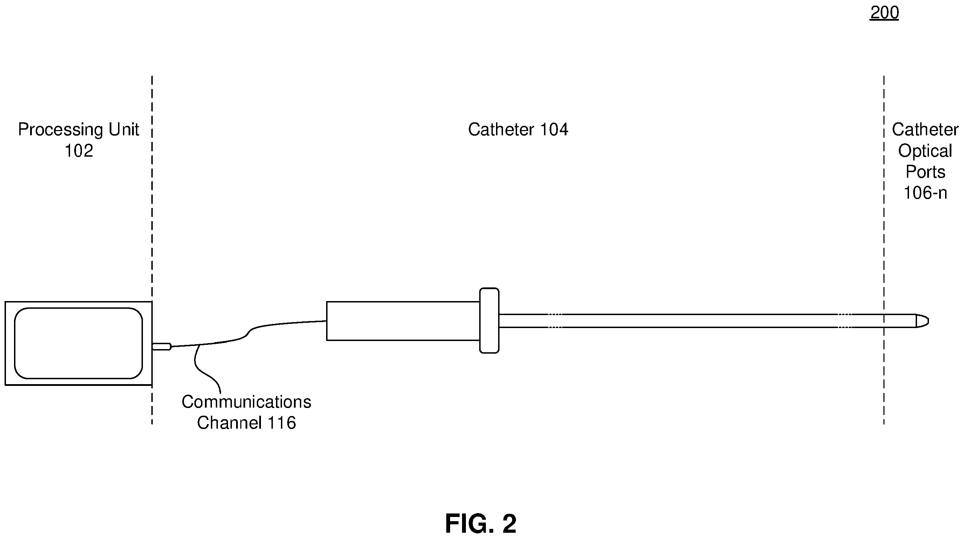

Catheter 104 is described with reference to FIG. 2. FIG. 2 is a diagram of a catheter 104, according to some aspects of the present disclosure.

Catheter 104 may be positioned towards a tissue of patient 114. Catheter 104 may be used to apply energy to a tissue of patient 114. Application of energy to a tissue of patient 114 may cause structural changes in the tissue. Structural changes may include lesion progression in a tissue.

Catheter 104 may house electrical or optical components. Electrical components may be used to provide signals to other components in catheter 104. Catheter 104 may include an optical source to generate a source beam of radiation (e.g., light) for optical evaluation for a tissue of patient 114. An optical source may include one or more laser diodes or light emitting diodes (LEDs). In some aspects, the beam of radiation generated by the optical source may have a wavelength within the infrared range. The beam of radiation generated by the optical source may output a beam at a single wavelength or it may output a beam at a range of different wavelengths. Optical measurement data may be measurements of the source beam of radiation.

Catheter 104 may include an optical transmission medium to guide the source beam of radiation throughout the catheter. The optical transmission medium may guide the source beam generated from the optical source towards catheter optical ports 106a-n. The source beam may interact with a tissue of patient 114. The optical transmission medium may guide the source beam back from a tissue of patient 114, through catheter optical ports 106-n, towards processing unit 102. In some aspects, the optical transmission medium may guide light in two directions. In some aspects, the optical transmission medium may be a single mode optical fiber, a multimode optical fiber, a plurality of optical fibers, or another photonic transmission medium.

Catheter 104 may also include an energy source to generate energy for tissue ablation. In some aspects, the energy source is an RF energy source, and the energy may propagate through catheter 104 via an electronic transmission medium. In other aspects, the energy source is another optical source, such as a laser energy source, to generate energy for tissue ablation. The optical energy may propagate through catheter 104 via an optical transmission medium.

In some aspects, catheter 104 may house electrical transmission media and optical transmission media in the form of a hybrid medium allowing for both electrical and optical signal propagation. Catheter 104 may include a protective cover that covers electrical and/or optical components.

As illustrated by systems 100 and 200, catheter 104 may include catheter optical ports 106a-n. Catheter optical ports 106a-n may be considered orifices at a tip of catheter 104. Catheter optical ports 106a-n may have been machined into the tip of catheter 104 or otherwise assembled into the tip of catheter 104. Catheter optical ports 106a-n may be distributed across and/or around the tip of catheter 104, resulting in distinct optical viewing directions. Catheter optical ports 106-n may transmit and collection light (e.g., optical measurement data) at various angles. Catheter optical ports 106a-n may also allow laser energy to be directed to a tissue of patient 114 for ablation at various angles.

Catheter 104 may include an irrigation pump to transfer fluid through the catheter and towards a tissue of patient 114. The fluid may be released through catheter optical ports 106a-n or another opening at the tip of the catheter. Fluid from the irrigation pump may cool the catheter or the tissue of patient 114. Fluid from the irrigation pump may also flush away debris during or after ablation.

Catheter 104 may include control elements so that a user can control the operation of the catheter. Catheter 104 may include a deflection control mechanism that controls a deflection angle of the catheter. Catheter 104 may include various buttons or switches to allow a user to control the application of ablation energy, when beams of radiation are transmitted, or when optical measurement data is acquired.

As illustrated by systems 100 and 200, catheter 104 may collect optical measurement data from catheter optical ports 106a-n in order to be processed by processing unit 102. Catheter 104 may interface with processing unit 102 through communications channel 116. Communications channel 116 may be wired, wireless, or a combination thereof. Communications channel 116 may include optical connections or electrical connections. Optical connections may include single mode optical fibers or multimode optical fibers that allow acquisition or transmission of optical signals, and electrical connections may include wiring, pins, or components used for supplying power and energy. Communications channel 116 may include any combination of Local Area Networks, Wide Area Networks, the Internet, etc. Control logic or data may be transmitted to and from catheter 104 via communications channel 116.

Catheter optical ports 106-n may be positioned towards a tissue of patient 114. Catheter optical ports 106a-n may be used to observe portions of a tissue of patient 114. Catheter optical ports 106-n may transmit and collect light (e.g., optical measurement data) at various angles.

Catheter optical ports 106-n may interface with optical transmission medium, used to guide the source beam of radiation throughout catheter 104 towards catheter optical ports 106a-n. The source beam may interact with a tissue of patient 114. Optical transmission medium may guide light reflected back from the tissue through catheter optical ports 106a-n, towards processing unit 102. The source beam of radiation may be from an optical source, including one or more laser diodes or LEDs. Optical measurement data may include measurements from the light reflected from the tissue and guided back to processing unit 102.

The location of catheter optical ports 106a-n respective to the tissue of the patient may change in accordance with a change in catheter 104 position. A line of sight of catheter optical ports 106a-n may be positioned at various incidence angles with respect to the tissue surface, including, for example and without limitation, at 0°, 29°, 45°, 62°, and 90°. Catheter optical ports 106a-n may be positioned to be in contact with a tissue of patient 114. The number of catheter optical ports 106a-n in contact with a tissue of patient 114 may be determined based on optical measurement data detected from light beams at the opening of catheter optical ports 106a-n. A catheter optical port can be switched on or off using processing unit 102 in order to receive optical measurement data at different times.

Returning to FIG. 1, processing unit 102 may interface with signal generator 110 in order to generate an energy signal for catheter 104 ablation of a tissue of patient 114. Signal generator 110 may be a device configured to generate radiofrequency (RF), cryogenic, laser, electroporation (e.g., pulsed electric field), or another energy signal for ablation. Signal generator 110 may interface with catheter 104 directly or through processing unit 102. For example, signal generator 110 may interface with processing unit 102 through communications channel 120. Communications channel 120 may be wired, wireless, or a combination thereof. Communications channel 120 may include optical connections or electrical connections. Optical connections may include single mode optical fibers or multimode optical fibers that allow acquisition or transmission of optical signals, and electrical connections may include wiring, pins, or components used for supplying power and energy. Control logic or data may be transmitted to and from signal generator 110 via communications channel 120.

The operation of processing unit 102 according to aspects of the invention will now be described. Processing unit 102 may include hardware, firmware, software, or any combination thereof to perform analysis of the optical measurement data or generate graphical representations. Processing unit 102 may be coupled to and receive data from an optical system, for example within catheter 104, to perform analysis of optical measurement data, to identify optical properties at various locations, or to generate graphical representations. The optical system may utilize low-coherence interferometry (LCI), optical coherence tomography (OCT), optical coherence refractometry (OCR), or other optical modalities to perform imaging and to obtain optical measurement data. Optical measurement data may be an OCT signal. Optical measurement data may be an OCR signal. Optical measurement data may be other data that would be appreciated by a person of ordinary skill in the art. Optical properties may include polarization and/or birefringence. Birefringence, or a loss of birefringence, may be correlated with necrosis and muscle fiber denaturation. Optical properties may be spectral information, and/or other properties that would be appreciated by a person of ordinary skill in the art.

Processing unit 102 may send display information (e.g., graphical representations) to user interface 108. The display information may include optical measurement data, optical properties, and/or information obtained therefrom (e.g., estimated lesion depth). The display information may also include contact information of catheter 104 (i.e., whether catheter 104 is sufficiently contacting the tissue) and/or other information useful for a user of user interface 108. Processing unit 102 may interface with user interface 108 through communications channel 112. Communications channel 112 may be wired, wireless, or a combination thereof. Communications channel 112 may include any combination of Local Area Networks, Wide Area Networks, the Internet, etc. Control logic or data may be transmitted to and from processing unit 102 via communications channel 112.

A graphical representation generated by processor 102 from the received optical measurement data and displayed on user interface 108 may illustrate or identify, for example, estimated lesion depth at a particular area of tissue. Estimated lesion depth may be a function of the ratio of ablation time over denaturation time. Estimated lesion depth may represent a height and a width of a lesion formed by the energy applied by signal generator 110 to a tissue of patient 114. The tissue of patient 114 may comprise myocardial tissue, cardiac muscle tissue, skeletal tissue, or the like. The tissue of patient 114 may be a portion of a tissue. Estimated lesion depth may be beneficial to monitor as high temperature thermal therapies may destroy tissues in the temperature range of 50 to 90 °C. At temperatures around 43 to 45 °C, irreversible cell damage, such as membrane collapse, protein denaturation, and mitochondrial dysfunction, may destroy cells after longer exposure times (e.g., 30-60 minutes). At higher temperatures, such as temperatures above 60 °C, rapid protein denaturation and cell death may often occur within seconds. An example study was conducted in order to develop a lesion depth estimation algorithm using optical property measurements from ablated tissue. In the study, tissue samples were excised from swine hearts, and an end of the catheter was perpendicularly positioned at the endocardial surface of the tissue using a micropositioner. The tissue samples included right atrial free wall, superior vena cava, left atrial roof, mitral annulus, and left atrial appendage. In aspects, graphical representations may be other representations that would be appreciated by a person of ordinary skill in the art.

FIGS. 3 and 5 further describe the generation of the graphical representations sent to user interface 108 by processing unit 102. Processing unit 102 may be a multi-core processor as illustrated by FIG. 3. For example, processing unit 102 may be an Intel^{®} x86-compatible multi-core processor. Processing unit 102 may contain two cores, core-1 304 and core-2 306. While two cores are illustrated in FIG. 3, one of skill in the art would recognize that processing unit 102 may contain more than two cores, such as 8 cores, 16 cores, 32 cores, etc., and that a higher number of cores can process more threads in parallel than a lower number of cores. Processing unit 102 may be or include a Central Processing Unit (CPU). A CPU may contain an arithmetic and logic unit to perform data processing. A CPU may contain a control unit to control the operation of the CPU. A CPU may contain a clock to supply timing signals. Each core of processing unit 102 may include a register and a cache. A register may provide storage for its respective core. A register may be large enough to hold an instruction. For example, a register may be 64 bits in length. Processing unit 102 may include a cache. A cache may store data for fast retrieval. Processing unit 102 may contain a bus. A bus may transfer messages between components of processing unit 102. Processing unit 102 may include a memory. A memory may be, for example, a Random Access Memory (RAM). Processing unit 102 may include an input/output (I/O) server. An I/O server may facilitate inputs to and outputs from processing unit 102.

As illustrated by system 300 of FIG. 3, a first catheter optical port 106-1 may receive a first optical measurement data 308 from a tissue of patient 114. Catheter optical port 106-1 may have a unique viewing angle of the tissue of patient 114. The unique viewing angle may view a first location of the tissue of patient 114. Processing unit 102 may receive first optical measurement data 308 through catheter 104 and communications channel 116. First catheter optical port 106-1 may be considered "active" or "open" when an optical switch at processing unit 102 allows first optical measurement data 308 to be received for processing. First catheter optical port 106-1 may be considered "inactive" or "closed" when an optical switch at processing unit 102 does not allow first optical measurement data 308 to be received for processing. Processing unit 102 may assign the received first optical measurement data 308 to a first available processing core (e.g., core-1 304).

The assigned processing core may implement a processing chain on the first optical measurement data 308 in order to obtain an optical property of the tissue of patient 114 at the first location and generate a graphical representation of the optical property. FIG. 6, further described below, illustrates a processing diagram 600 that includes an example processing chain 610. Processing chain 610 may obtain first optical measurement data 308 related to an optical property at the first location. First optical measurement data 308 may first be analyzed to determine the optical property. The optical property may then be used to generate a first graphical representation of the optical property for display on a graphical user interface, such as graphical user interface 108. In an aspect, the first graphical representation may indicate an estimated lesion depth at the first location.

Processing chain 610 may consistently require a certain amount of time after receiving measurement data for completion of the process and outputting of the graphical representation. FIG. 5 illustrates an example timing diagram according to aspects of the present invention. FIG. 5 illustrates the processing activity of each core of processing unit 102. Given the two-core example of FIG. 3, FIG. 5 shows the processing activity of the two cores. The timeline of processing core-1 304 is illustrated at the top of FIG. 5; the timeline of processing core-2 306 is illustrated at the bottom of FIG. 5. For processing core-1 304, the processing chain begins once first optical measurement data 308 has been received. The receipt of first optical measurement data 308 indicates a time 0 at which processing begins. In the timing diagram of FIG. 5, this time 0 starts at 0 ms.

The processing chain, such as processing chain 610, operating on processing core-1 304 takes a certain amount of time to complete its execution. The amount of time to completion is illustrated in FIG. 5 as T_{R}, such that processing chain 610 is active from time 0-T_{R}. T_{R} is determined by both the speed of the processor and the number of calculations or other functions the processor needs to execute in order to complete the processing chain, such as the time it takes processing chain 610 to calculate the optical property and generate a graphical representation thereof. In some aspects, T_{R} may be obtained by benchmarking a certain processing chain using sample data with the processor used to execute the processing chain. In an aspect, T_{R} for processing chain 610 is 300 milliseconds. In another aspect, T_{R} is between 200 and 400 milliseconds. In another aspect, T_{R} is less than 300 ms. Once a graphical representation has been generated, processing unit 102 may store the first graphical representation until a predefined interval has passed for updating user interface 108.

After a first cycle of data collection from first optical port 106-1 has completed, and at the time processing chain 610 begins processing first optical measurement data 308, an optical switch at processing unit 102 closes the connection with first catheter optical port 106-1 and opens a connection with a second catheter optical port 106-2, so that data from second catheter optical port 106-2 may be collected by processing unit 102. This switch may occur at a predetermined time period after data collection from first catheter optical port 106-1 begins. According to some aspects, the predetermined time period may be selected based on the amount of time it takes to obtain sufficient data from which an optical property corresponding to optical port 106-1's field of view may be determined. For example, the predetermined time period may be 2 milliseconds (as illustrated in FIG. 5). In another example, the predetermined time period may be 1 millisecond. In yet another example, the predetermined time period may be another time period less than or more than 2 milliseconds.

As shown in FIG. 3, second catheter optical port 106-2 may receive a second optical measurement data 310 from a tissue of patient 114. Second catheter optical port 106-2 may be activated to receive the second optical measurement data 310 from a tissue of patient 114 at the predetermined time period after first catheter optical port 106-1 is activated to receive the first optical measurement data 308. In order to receive the second optical measurement data 310 from a second catheter optical port 106-2, processing unit 102 may switch an input from the first catheter optical port 106-1 to the second catheter optical port 106-2 at or after the predetermined time.

Catheter optical port 106-2 may have a unique viewing angle of the tissue of patient 114. The unique viewing angle may view a second location of the tissue of patient 114. Processing unit 102 may receive the second optical measurement data 310 through catheter 104 and communications channel 116. Processing unit 102 may assign the second optical measurement data 310 to a second available processing core (e.g., core-2 306).

Turning to FIG. 5, activity of processing core-2 306 is illustrated at the bottom of FIG. 5. As mentioned above, an input from second catheter optical port 106-2 may be activated or opened at the time processing core-1 304 begins to execute processing chain 610 on data from first catheter optical port 106-1. Once processing unit 102 switches to second catheter optical port 106-2, it may take some time to receive second optical measurement data 310 from second catheter optical port 106-2. Once second optical measurement data 310 is received, processing chain 610 on processing core-2 306 can begin processing second optical measurement data 310.

As discussed above, FIG. 5 illustrates a timing diagram in which the time it takes to receive the optical measurement data is 2 ms. This means that processing core-2 306 is not active until that time. While processing chain 610 on processing core-1 304 begins operation on optical measurement data 308 at time 0 (shown as 0 ms in FIG. 5) and completes its process at time T_{R}, processing chain 610 on processing core-2 306 is delayed until optical measurement data 310 is received (shown as 2 ms in FIG. 5). Because processing chain 610 takes time T_{R} to complete its execution, processing chain 610 on processing core-2 306 begins operation on optical measurement data 310 at 2 ms and completes operation at time T_{R}+2 ms.

While the first available processing core is implementing processing chain 610 on the first optical measurement data 308, the second available processing core is implementing processing chain 610 on the second optical measurement data 310 with an offset equivalent to the predetermined time. The assigned processing core may implement processing chain 610 on the second optical measurement data 310 in order to obtain an optical property of the tissue of patient 114 at the second location. Processing chain 610 may generate a second graphical representation from the optical property at the second location. In an aspect, the second graphical representation may be an estimated lesion depth at the second location.

In some aspects, the graphical representations are displayed on a graphical user interface as soon as they are generated by their respective processing cores. In such aspects, an overall field of view of the optical catheter may be updated incrementally. In other aspects, the graphical representations are displayed collectively on a graphical user interface once graphical representation corresponding to all optical ports are generated. In such aspects, an overall field of view of the optical catheter may be updated at one time. For example, once a second graphical representation has been generated, processing unit 102 may collect the first graphical representation and the second graphical representation to display on user interface 108. The time that has passed since the last display update on user interface 108 may correspond to a predefined interval.

The predefined interval may be the time to implement processing chain 610 on the first optical measurement data 308 and to generate the first graphical representation (T_{R}, e.g., 300 milliseconds), while in parallel, the time to receive the second optical measurement data 310 (the predetermined time, e.g., 2 milliseconds), and the time to implement processing chain 610 on the second optical measurement data 310 and to generate the second graphical representation (T_{R+2}). Because the available processing cores are implementing processing chain 610 on the first 308 and second optical measurement data 310 in parallel (e.g., simultaneously with a 2 millisecond delay for the second), processing chain 102 may be able to update the display of user interface 108 at a predefined interval that is a multiple of the predetermined time and the number of catheter optical ports 106-n. For example, if the predetermined time is 2 milliseconds and there are two catheter optical ports 106-1 and 106-2, then processing unit 102 may display the first graphical representation and the second graphical representation on user interface 108 every 4 milliseconds. In another example, if the predetermined time is 2 milliseconds and there were seven catheter optical ports 106-n, then processing unit 102 may display seven graphical representations on user interface 108 every 14 milliseconds.

Referring now to FIG. 6, processing cores 304 or 306 undergo processing in processing unit 102 as illustrated by processing diagram 600. Processing, including data transfer, may be optimized in different software abstract layers in order to maintain data integrity while improving refresh time of user interface 108. Processing diagram 600 includes optical measurement data acquisition 602, hardware abstraction layer (HAL) 604, information processing library (IPL) 606, and ablation representation layer (ARL) 608. IPL 606 and ARL 608 may make up processing chain 610.

Optical measurement data acquisition 602 may include receiving a first optical measurement data 308 from catheter optical port 106-1 via catheter 104 and communications channel 116. Optical measurement data acquisition 602 may include receiving a second optical measurement data 310 from catheter optical port 106-2 via catheter 104 and communications channel 116.

HAL 604 may receive optical measurement data, interface between the firmware/hardware, and control data flow.

IPL 606 may receive optical measurement data from HAL 604 and provide optimization algorithms in order to optimize the quality of information. IPL 606 may rearrange optical measurement data 308 or 310, remove glitches in optical measurement data 308 or 310, conduct a Hilbert transform on optical measurement data 308 or 310, remove phase noise from optical measurement data 308 or 310, linearize phase of optical measurement data 308 or 310, compensate for polarization mode of optical measurement data 308 or 310, conduct a Fourier transform on optical measurement data 308 or 310, and/or perform other processing of optical measurement data 308 or 310. IPL 606 may perform processing in order to identify optical properties at various tissue locations of patient 114.

ARL 608 may receive processed optical measurement data from IPL 606 and may provide graphical representations of the data to user interface 108. ARL 608 may conduct reference channel processing, detect and categorize tissue, estimate birefringence, estimate lateral speed of catheter 104, or perform other analytics of optical measurement data 308 or 310. ARL 608 may perform analytics in order to generate a graphical representation to display on user interface 108.

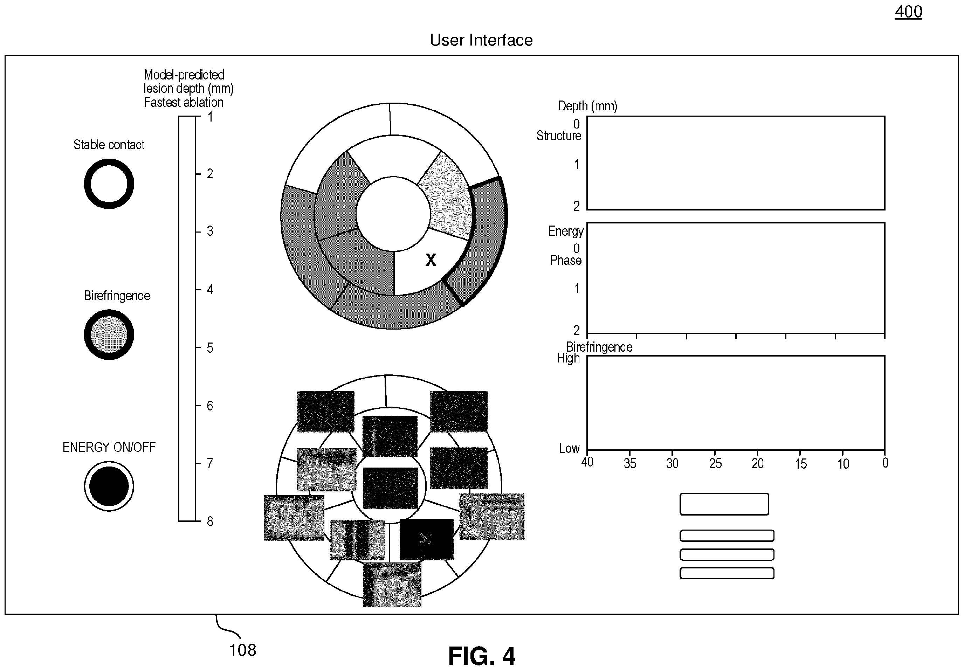

User interface 108 is described with reference to FIG 4. FIG. 4 is a diagram of a user interface 108, according to some aspects of the present disclosure.

User interface 108 may be a display. User interface 108 may include a graphical user interface (GUI). Illustration 400 shows an example GUI. The GUI may include multiple sections, such as sections 402 and 404, showing different information related to catheter 104. In the aspect of FIG. 4, section 402 is a contact map showing the amount of contact between a patient 114's tissue and each of catheter optical ports 106a-n, and which beams from catheter optical ports 106a-n are in operation. For example, a white colored section may indicate a strong, stable contact between catheter 104 and tissue of patient 114, a lighter colored section may indicate a minimal or intermediate contact between catheter 104 and tissue of patient 114, and darker sections may indicate no contact between catheter 104 and tissue of patient 114 and/or that the optical port is turned off. By viewing the contact map shown in section 402 of the GUI, a user can quickly determine which portions of the catheter have stable contact with the tissue, and which do not.

Section 404 of the GUI may include a plurality of tiles, each tile showing an optical readout for a respective catheter optical port 106a-n. The number of tiles displayed corresponds to the number of catheter optical ports 106a-n. Each tile may represent an image resulting from processing, by processing unit 102, the optical measurement data from a respective optical port 106a-n. Individual tiles may be switched on or off, and thus may appear or disappear, based on activity. Section 404 of the GUI may be arranged such that the locations of the tiles are analogous to or otherwise indicative of the positions of the optical ports on the catheter tip. By viewing the tiles in section 404 of the GUI, a user can quickly see the collective data from across the catheter tip.

In some aspects, as shown in illustration 400, the GUI may also include one or more charts illustrating ablation energy data, birefringence data, phase data, and/or estimated lesion depth data.

The GUI may include a panel or indicator showing the occurrence of stable contact between catheter 104 or catheter optical ports 106-n and tissue of patient 114, loss in birefringence, status of the ablation energy (e.g., on/off), and estimated lesion depth.

The GUI may include a button or a text box allowing user selection or customization of parameters selected for ablation or for operating catheter 104 during ablation.

User interface 108 may refresh the graphical representations shown at a predefined interval. The predefined interval may correspond to a predetermined amount of time needed to receive optical measurement data from each of catheter optical ports 106a-n. For example, if the predetermined time of receiving optical measurement data for a single catheter optical port is 2 milliseconds, and there are seven catheter optical ports 106a-n, then user interface 108 may display updated graphical representations every 14 milliseconds. In another example, if the predetermined amount of time needed to receive optical measurement data for a single catheter optical port is 1 millisecond and there are five catheter optical ports 106a-n, then user interface 108 may display graphical representations every 5 milliseconds. The graphical representations may include, for example, an estimated lesion depth.

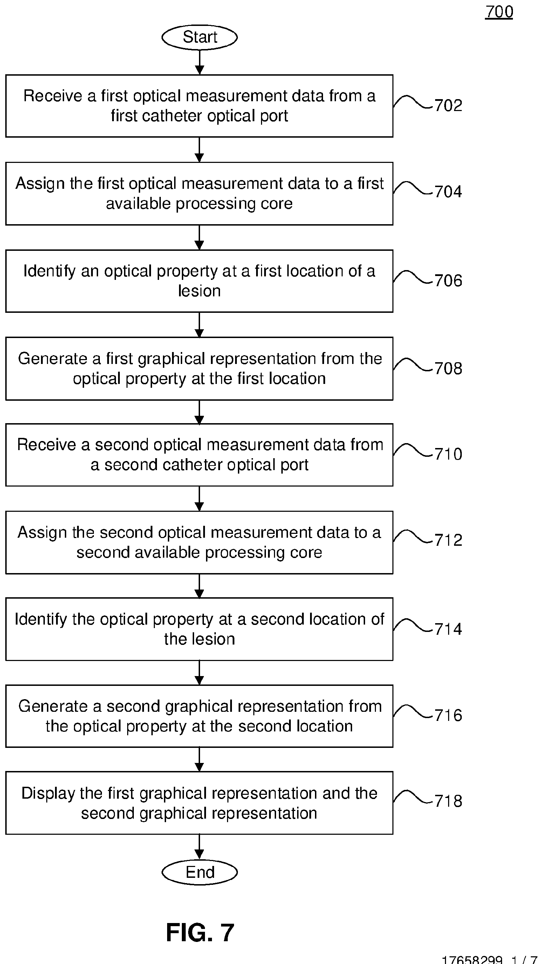

FIG. 7 is a flowchart for a method 700 for assessing ablation lesions in realtime, according to an aspect of the invention. Method 700 can be performed by processing logic that can comprise hardware (e.g., circuitry, dedicated logic, programmable logic, microcode, etc.), software (e.g., instructions executing on a processing device), or a combination thereof. It is to be appreciated that not all steps may be needed to perform the disclosure provided herein. Further, some of the steps may be performed simultaneously, or in a different order than shown in FIG. 7, as will be understood by a person of ordinary skill in the art.

Method 700 shall be described with reference to FIGS. 1, 3, and 6. However, method 700 is not limited to that example aspect.

In 702, a first optical measurement data is received from a first catheter optical port. In an example, the first optical measurement data 308 may first be transmitted from the first catheter optical port 106-1 to a hardware abstraction layer 604. HAL 604 may receive optical measurement data, interface between the firmware/hardware, and control data flow. Processing unit 102 may then receive the first optical measurement data 308 from the hardware abstraction layer 604.

In 704, processing of the first optical measurement data is assigned to a first available processing core of the processing unit. In an example, processing unit 102 assigns the first optical measurement data 308 received in 702 to a first available processing core (e.g., core-1 304) in processing unit 102.

In 706, an optical property at a first tissue location, which may correspond to a lesion, is identified. In an example, the first available processing core from 704 identifies an optical property at a first location of a lesion on tissue of patient 114 from the first optical measurement data 308 received in 702.

In some aspects, the first available processing core from 704 may identify an optical property at a first location by implementing processing chain 610 on the first optical measurement data 308 received in 702. Processing chain 610 may include an information processing library (IPL) 606. IPL 606 may receive optical measurement data from HAL 604 and provide optimization algorithms in order to optimize the quality of information. IPL 606 may rearrange optical measurement data 308, remove glitches in optical measurement data 308, conduct a Hilbert transform on optical measurement data 308, remove phase noise from optical measurement data 308, linearize phase of optical measurement data 308, compensate for polarization mode of optical measurement data 308, conduct a Fourier transform on optical measurement data 308, and/or perform other processing of optical measurement data 308. In some aspects, IPL 606 may perform processing in order to identify an optical property at a first location of a lesion of patient 114.

The optical property identified in 706 may be birefringence. Birefringence, or a loss of birefringence, may be correlated with necrosis and muscle fiber denaturation.

In 708, a first graphical representation is generated from the optical property at the first location. In an example, the first available processing core from 704 generates a first graphical representation from the optical property at the first location identified in 706.

The first available processing core from 704 may generate a first graphical representation from the optical property at the first location by implementing processing chain 610 on the optical property identified by the IPL. Processing chain 610 may include an ablation representation layer (ARL) 608. ARL 608 may receive processed optical measurement data from IPL 606 and may provide data representations to user interface 108. ARL 608 may conduct reference channel processing, detect and categorize tissue, estimate birefringence, estimate lateral speed of catheter 104, and/or perform other analytics of optical measurement data 308. In some aspects, ARL 608 may perform analytics in order to generate a first graphical representation to display on user interface 108.

The first graphical representation may be an estimated lesion depth. Estimated lesion depth may be a function of the ratio of ablation time over denaturation time. Estimated lesion depth may represent a height and a width of a lesion formed by the energy applied by signal generator 110 to a tissue of patient 114.

In 710, a second optical measurement data is received from a second catheter optical port. In an example, processing unit 102 receives a second optical measurement data 310 from a second catheter optical port 106-2.

The processing unit 102 may receive the second optical measurement data 310 from a second catheter optical port 106-2 at a predetermined time after receiving a first optical measurement data 308 in 702. For example, the predetermined time may be 2 milliseconds. In another example, the predetermined time may be 1 millisecond. In yet another example, the predetermined time may be another time period less than 2 milliseconds.

In order to receive the second optical measurement data 310 from a second catheter optical port 106-2, processing unit 102 may switch an input from the first catheter optical port 106-1 to the second catheter optical port 106-2 after the predetermined time has passed.

The second optical measurement data 310 may first be transmitted from the second catheter optical port 106-2 to a hardware abstraction layer 604. HAL 604 may receive optical measurement data, interface between the firmware/hardware, and control data flow. Processing unit 102 may then receive the second optical measurement data 310 from the hardware abstraction layer 604.

In 712, the second optical measurement data is assigned to a second available processing core. In an example, processing unit 102 assigns the second optical measurement data 310 received in 710 to a second available processing core (e.g., core-2 306) in processing unit 102.

In 714, the optical property at a second location of the tissue, which may be a lesion, is identified. In an example, the second available processing core from 712 identifies the optical property at a second location of a lesion on tissue of patient 114 from the second optical measurement data 310 received in 710.

The second available processing core from 712 may identify an optical property at a second location by implementing processing chain 610 on the second optical measurement data 310 received in 710. Processing chain 610 may include an information processing library (IPL) 606. IPL 606 may receive optical measurement data from HAL 604 and provide optimization algorithms in order to optimize the quality of information. IPL 606 may rearrange optical measurement data 310, remove glitches in optical measurement data 310, conduct a Hilbert transform on optical measurement data 310, remove phase noise from optical measurement data 310, linearize phase of optical measurement data 310, compensate for polarization mode of optical measurement data 310, conduct a Fourier transform on optical measurement data 310, and/or perform other processing of optical measurement data 310. In some aspects, IPL 606 may perform processing in order to identify an optical property at a second location of a lesion of patient 114.

In some aspects, the optical property at a second location identified in 714 may be birefringence. Birefringence, or a loss of birefringence, may be correlated with necrosis and muscle fiber denaturation.

In 716, a second graphical representation is generated from the optical property at the second location. In an example, the second available processing core from 712 generates a second graphical representation from the optical property at the second location identified in 714.

The second available processing core from 712 may generate a second graphical representation from the optical property at the second location by implementing processing chain 610 on the optical property identified by the IPL. Processing chain 610 may include an ablation representation layer (ARL) 608. ARL 608 may receive processed optical measurement data from IPL 606 and may provide data representations to user interface 108. ARL 608 may conduct reference channel processing, detect and categorize tissue, estimate birefringence, estimate lateral speed of catheter 104, and/or perform other analytics of optical measurement data 310. In some aspects, ARL 608 may perform analytics in order to generate a second graphical representation to display on user interface 108.

In some aspects, the second graphical representation may be an estimated lesion depth. Estimated lesion depth may be a function of the ratio of ablation time over denaturation time. Estimated lesion depth may represent a height and a width of a lesion formed by the energy applied by signal generator 110 to a tissue of patient 114.

In 718, the first graphical representation and the second graphical representation are displayed. In some aspects, processing unit 102 displays the first graphical representation from 708 and the second graphical representation from 716 on user interface 108. In some aspects, processing unit 102 displays the first graphical representation from 708 and the second graphical representation from 716 at the same time, upon completion of second graphical representation from 716. In other aspects, processing unit 102 displays the first graphical representation upon its completion in 708, and then updates user interface 108 with the display of the second graphical representation upon its completion in 716.

In some aspects, the first graphical representation from 708 and the second graphical representation from 716 may be displayed on user interface 108 at a predefined interval. The predefined interval may be the time to implement processing chain 610 on the first optical measurement data 308 and to generate the first graphical representation (T_{R}, e.g., 300 milliseconds), while in parallel, the time to receive the second optical measurement data 310 (the predetermined time, e.g., 2 milliseconds), and the time to implement processing chain 610 on the second optical measurement data 310 and to generate the second graphical representation (T_{R+2}). Because the available processing cores are implementing processing chain 610 on the first optical measurement data 308 and second optical measurement data 310 in parallel (e.g., with a 2 millisecond offset), processing chain 102 may be able to update the display of user interface 108 at a predefined interval that is a multiple of the predetermined time and the number of catheter optical ports 106-n. For example, if the predetermined time is 2 milliseconds and there are two catheter optical ports 106-1 and 106-2, then processing unit 102 may display the first graphical representation and the second graphical representation on user interface 108 every 4 milliseconds. In another example, if the predetermined time is 2 milliseconds and there were seven catheter optical ports 106-n, then processing unit 102 may display seven graphical representations on user interface 108 every 14 milliseconds.

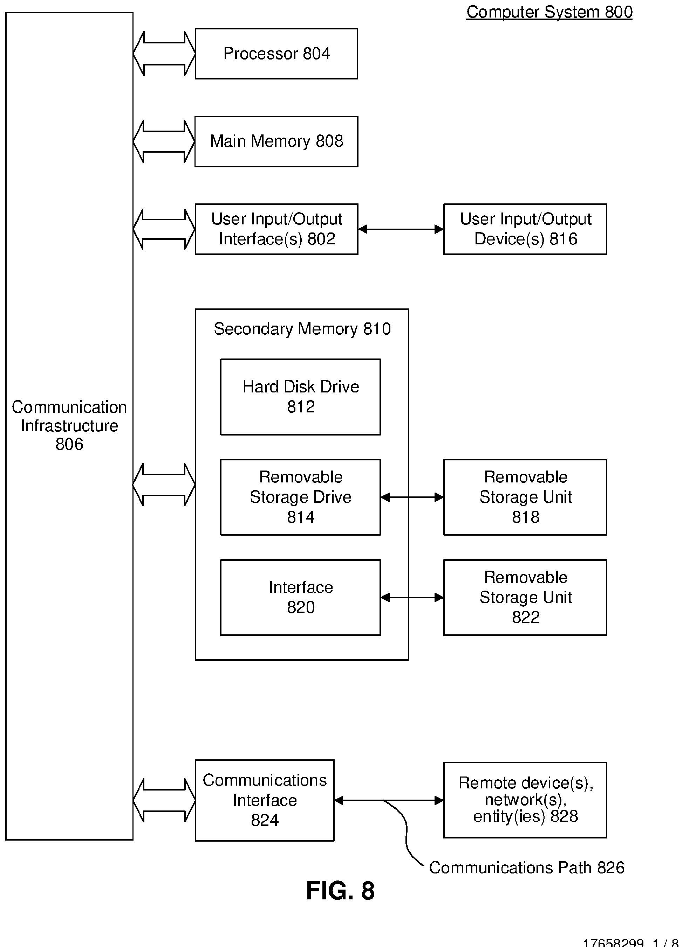

Various aspects can be implemented, for example, using one or more computer systems, such as computer system 800 shown in FIG. 8. Computer system 800 can be used, for example, to implement method 700 of FIG. 7. For example, computer system 800 can receive optical measurement data, identify optical properties, and generate graphical representations. Computer system 800 can also implement a processing chain in multiple processing cores in order to decrease the time needed to display graphical representations on a user interface, according to some aspects. Computer system 800 can be any computer capable of performing the functions described herein.

Computer system 800 can be any well-known computer capable of performing the functions described herein.

Computer system 800 includes one or more processors (also called central processing units, or CPUs), such as a processor 804. Processor 804 is connected to a communication infrastructure or bus 806.

One or more processors 804 may each be a graphics processing unit (GPU). In an aspect, a GPU is a processor that is a specialized electronic circuit designed to process mathematically intensive applications. The GPU may have a parallel structure that is efficient for parallel processing of large blocks of data, such as mathematically intensive data common to computer graphics applications, images, videos, etc.

Computer system 800 also includes user input/output device(s) 816, such as monitors, keyboards, pointing devices, etc., that communicate with communication infrastructure 806 through user input/output interface(s) 802.

Computer system 800 also includes a main or primary memory 808, such as random access memory (RAM). Main memory 808 may include one or more levels of cache. Main memory 808 has stored therein control logic (i.e., computer software) and/or data.

Computer system 800 may also include one or more secondary storage devices or memory 810. Secondary memory 810 may include, for example, a hard disk drive 812 and/or a removable storage device or drive 814. Removable storage drive 814 may be a floppy disk drive, a magnetic tape drive, a compact disk drive, an optical storage device, tape backup device, and/or any other storage device/drive.

Removable storage drive 814 may interact with a removable storage unit 818. Removable storage unit 818 includes a computer usable or readable storage device having stored thereon computer software (control logic) and/or data. Removable storage unit 818 may be a floppy disk, magnetic tape, compact disk, DVD, optical storage disk, and/ any other computer data storage device. Removable storage drive 814 reads from and/or writes to removable storage unit 818 in a well-known manner.

According to an exemplary aspect, secondary memory 810 may include other means, instrumentalities or other approaches for allowing computer programs and/or other instructions and/or data to be accessed by computer system 800. Such means, instrumentalities or other approaches may include, for example, a removable storage unit 822 and an interface 820. Examples of the removable storage unit 822 and the interface 820 may include a program cartridge and cartridge interface (such as that found in video game devices), a removable memory chip (such as an EPROM or PROM) and associated socket, a memory stick and USB port, a memory card and associated memory card slot, and/or any other removable storage unit and associated interface.

Computer system 800 may further include a communication or network interface 824. Communication interface 824 enables computer system 800 to communicate and interact with any combination of remote devices, remote networks, remote entities, etc. (individually and collectively referenced by reference number 828). For example, communication interface 824 may allow computer system 800 to communicate with remote devices 828 over communications path 826, which may be wired and/or wireless, and which may include any combination of LANs, WANs, the Internet, etc. Control logic and/or data may be transmitted to and from computer system 800 via communication path 826.

In an aspect, a tangible, non-transitory apparatus or article of manufacture comprising a tangible, non-transitory computer useable or readable medium having control logic (software) stored thereon is also referred to herein as a computer program product or program storage device. This includes, but is not limited to, computer system 800, main memory 808, secondary memory 810, and removable storage units 818 and 822, as well as tangible articles of manufacture embodying any combination of the foregoing. Such control logic, when executed by one or more data processing devices (such as computer system 800), causes such data processing devices to operate as described herein.

Based on the teachings contained in this disclosure, it will be apparent to persons skilled in the relevant art(s) how to make and use aspects of this disclosure using data processing devices, computer systems and/or computer architectures other than that shown in FIG. 8. In particular, aspects can operate with software, hardware, and/or operating system implementations other than those described herein.

It is to be appreciated that the Detailed Description section, and not any other section, is intended to be used to interpret the claims. Other sections can set forth one or more but not all exemplary aspects as contemplated by the inventor(s), and thus, are not intended to limit this disclosure or the appended claims in any way.

While this disclosure describes exemplary aspects for exemplary fields and applications, it should be understood that the disclosure is not limited thereto. Other aspects and modifications thereto are possible, and are within the scope and spirit of this disclosure. For example, and without limiting the generality of this paragraph, aspects are not limited to the software, hardware, firmware, and/or entities illustrated in the figures and/or described herein. Further, aspects (whether or not explicitly described herein) have significant utility to fields and applications beyond the examples described herein.

Aspects have been described herein with the aid of functional building blocks illustrating the implementation of specified functions and relationships thereof. The boundaries of these functional building blocks have been arbitrarily defined herein for the convenience of the description. Alternate boundaries can be defined as long as the specified functions and relationships (or equivalents thereof) are appropriately performed. Also, alternative aspects can perform functional blocks, steps, operations, methods, etc. using orderings different than those described herein.

References herein to "one aspect," "an aspect," "an example aspect," or similar phrases, indicate that the aspect described can include a particular feature, structure, or characteristic, but every aspect can not necessarily include the particular feature, structure, or characteristic. Moreover, such phrases are not necessarily referring to the same aspect. Further, when a particular feature, structure, or characteristic is described in connection with an aspect, it would be within the knowledge of persons skilled in the relevant art(s) to incorporate such feature, structure, or characteristic into other aspects whether or not explicitly mentioned or described herein. Additionally, some aspects can be described using the expression "coupled" and "connected" along with their derivatives. These terms are not necessarily intended as synonyms for each other. For example, some aspects can be described using the terms "connected" and/or "coupled" to indicate that two or more elements are in direct physical or electrical contact with each other. The term "coupled," however, can also mean that two or more elements are not in direct contact with each other, but yet still co-operate or interact with each other.

The breadth and scope of this disclosure should not be limited by any of the above-described exemplary aspects, but should be defined only in accordance with the following claims and their equivalents.

## Claims

1. A computer implemented method for assessing ablation lesions in realtime, comprising:
receiving, by at least one processor, a first optical measurement data from a first catheter optical port;
assigning, by the at least one processor, the first optical measurement data to a first available processing core in a processing unit;
directing, by the at least one processor, the first available processing core to implement a processing chain on the first optical measurement data in order to identify an optical property at a first location of a lesion;
generating, by the at least one processor, a first graphical representation from the optical property at the first location;
receiving, by the at least one processor, a second optical measurement data from a second catheter optical port after a predetermined time of receiving the first optical measurement data;
assigning, by the at least one processor, the second optical measurement data to a second available processing core in the processing unit;
directing, by the at least one processor, the second available processing core to implement the processing chain on the second optical measurement data in order to identify the optical property at a second location of the lesion;
generating, by the at least one processor, a second graphical representation from the optical property at the second location; and
displaying, by the at least one processor, the first graphical representation and the second graphical representation on a user interface at a predefined interval.

2. The computer implemented method of claim 1, further comprising:
transmitting, by the at least one processor, the first optical measurement data to a hardware abstraction layer that interfaces between the first catheter optical port and the processing unit.

3. The computer implemented method of claim 1, further comprising:
transmitting, by the at least one processor, the second optical measurement data to a hardware abstraction layer that interfaces between the second catheter optical port and the processing unit.

4. The computer implemented method of claim 1, wherein the processing chain comprises at least one of:
rearranging the first optical measurement data;
removing a glitch in the first optical measurement data;
conducting a Hilbert transform on the first optical measurement data;
removing a phase noise from the first optical measurement data;
linearizing a phase of the first optical measurement data;
compensating for a polarization mode of the first optical measurement data; or
conducting a Fourier transform on the first optical measurement data.

5. The computer implemented method of claim 1, wherein the processing chain comprises at least one of:
rearranging the second optical measurement data;
removing a glitch in the second optical measurement data;
conducting a Hilbert transform on the second optical measurement data;
removing a phase noise from the second optical measurement data;
linearizing a phase of the second optical measurement data;
compensating for a polarization mode of the second optical measurement data; or
conducting a Fourier transform on the second optical measurement data.

6. The computer implemented method of claim 1, wherein the optical property is birefringence.

7. The computer implemented method of claim 1, wherein the first graphical representation is an estimated lesion depth.

8. The computer implemented method of claim 1, wherein the second graphical representation is an estimated lesion depth.

9. The computer implemented method of claim 1, further comprising:
switching, by the at least one processor, an input of the at least one processor from the first catheter optical port to the second catheter optical port after the predetermined time.

10. The computer implemented method of claim 1, wherein the predetermined time is 2 milliseconds.

11. The computer implemented method of claim 1, wherein the predetermined time is 1 millisecond.

12. A system for assessing ablation lesions in realtime, comprising:
a catheter comprising a first catheter optical port and a second catheter optical port;
a computing device coupled to the catheter, the computing device comprising:
a processor, wherein the processor further comprises a processing unit; and
a memory, wherein the memory contains instructions stored thereon that when executed by the processor cause the computing device to:
receive a first optical measurement data from the first catheter optical port;
assign the first optical measurement data to a first available processing core in the processing unit;
direct the first available processing core to implement a processing chain on the first optical measurement data in order to identify an optical property at a first location of a lesion;
generate a first graphical representation from the optical property at the first location;
receive a second optical measurement data from the second catheter optical port after a predetermined time of receiving the first optical measurement data;
assign the second optical measurement data to a second available processing core in the processing unit;
direct the second available processing core to implement the processing chain on the second optical measurement data in order to identify the optical property at a second location of the lesion;
generate a second graphical representation from the optical property at the second location; and
display the first graphical representation and the second graphical representation on a user interface at a predefined interval;
and the user interface coupled to the computing device.

13. The system of claim 12, wherein the memory contains further instructions stored thereon that when executed by the processor cause the computing device to:
transmit the first optical measurement data to a hardware abstraction layer that interfaces between the first catheter optical port and the processing unit.

14. The system of claim 12, wherein the memory contains further instructions stored thereon that when executed by the processor cause the computing device to:
transmit the second optical measurement data to a hardware abstraction layer that interfaces between the second catheter optical port and the processing unit.

15. The system of claim 12, wherein the processing chain comprises at least one of:
rearrange the first optical measurement data;
remove a glitch in the first optical measurement data;
conduct a Hilbert transform on the first optical measurement data;
remove a phase noise from the first optical measurement data;
linearize a phase of the first optical measurement data;
compensate for a polarization mode of the first optical measurement data; or
conduct a Fourier transform on the first optical measurement data.

16. The system of claim 12, wherein the processing chain comprises at least one of:
rearrange the second optical measurement data;
remove a glitch in the second optical measurement data;
conduct a Hilbert transform on the second optical measurement data;
remove a phase noise from the second optical measurement data;
linearize a phase of the second optical measurement data;
compensate for a polarization mode of the second optical measurement data; or
conduct a Fourier transform on the second optical measurement data.

17. The system of claim 12, wherein the optical property is birefringence.

18. The system of claim 12, wherein the first graphical representation is an estimated lesion depth.

19. The system of claim 12, wherein the second graphical representation is an estimated lesion depth.

20. The system of claim 12, wherein the memory contains further instructions stored thereon that when executed by the processor cause the computing device to:
switch an input of the processor from the first catheter optical port to the second catheter optical port after the predetermined time.

21. The system of claim 12, wherein the predetermined time is 2 milliseconds.

22. The system of claim 12, wherein the predetermined time is 1 millisecond.

23. A non-transitory computer-readable medium having instructions stored thereon that, when executed by at least one computing device, cause the at least one computing device to perform operations comprising:
receiving a first optical measurement data from a first catheter optical port;
assigning the first optical measurement data to a first available processing core in a processing unit;
directing the first available processing core to implement a processing chain on the first optical measurement data in order to identify an optical property at a first location of a lesion;
generating a first graphical representation from the optical property at the first location;
receiving a second optical measurement data from a second catheter optical port after a predetermined time of receiving the first optical measurement data;
assigning the second optical measurement data to a second available processing core in the processing unit;
directing the second available processing core to implement the processing chain on the second optical measurement data in order to identify the optical property at a second location of the lesion;
generating a second graphical representation from the optical property at the second location; and
displaying the first graphical representation and the second graphical representation on a user interface at a predefined interval.

24. The non-transitory computer-readable medium of claim 23, wherein the operations further comprise:
transmitting the first optical measurement data to a hardware abstraction layer that interfaces between the first catheter optical port and the processing unit.

25. The non-transitory computer-readable medium of claim 23, wherein the operations further comprise:
transmitting the second optical measurement data to a hardware abstraction layer that interfaces between the second catheter optical port and the processing unit.

26. The non-transitory computer-readable medium of claim 23, wherein the processing chain comprises at least one of:
rearranging the first optical measurement data;
removing a glitch in the first optical measurement data;
conducting a Hilbert transform on the first optical measurement data;
removing a phase noise from the first optical measurement data;
linearizing a phase of the first optical measurement data;
compensating for a polarization mode of the first optical measurement data; or
conducting a Fourier transform on the first optical measurement data.

27. The non-transitory computer-readable medium of claim 23, wherein the processing chain comprises at least one of:
rearranging the second optical measurement data;
removing a glitch in the second optical measurement data;
conducting a Hilbert transform on the second optical measurement data;
removing a phase noise from the second optical measurement data;
linearizing a phase of the second optical measurement data;
compensating for a polarization mode of the second optical measurement data; or
conducting a Fourier transform on the second optical measurement data.

28. The non-transitory computer-readable medium of claim 23, wherein the optical property is birefringence.

29. The non-transitory computer-readable medium of claim 23, wherein the first graphical representation is an estimated lesion depth.

30. The non-transitory computer-readable medium of claim 23, wherein the second graphical representation is an estimated lesion depth.

31. The non-transitory computer-readable medium of claim 23, wherein the operations further comprise:
switching an input of the processing unit from the first catheter optical port to the second catheter optical port after the predetermined time.

32. The non-transitory computer-readable medium of claim 23, wherein the predetermined time is 2 milliseconds.

33. The non-transitory computer-readable medium of claim 23, wherein the predetermined time is 1 millisecond.
